(19) Europäisches Patentamt
European Patent Office
Office européen des brevets

(11) **EP 3 178 471 B1**

(12) **EUROPEAN PATENT SPECIFICATION**

(45) Date of publication and mention
of the grant of the patent:
**19.06.2019 Bulletin 2019/25**

(51) Int Cl.:
**A61K 9/16** *(2006.01)* **A61K 31/519** *(2006.01)*
**A61P 25/18** *(2006.01)*

(21) Application number: **15828945.4**

(22) Date of filing: **01.07.2015**

(86) International application number:
**PCT/KR2015/006771**

(87) International publication number:
**WO 2016/021835 (11.02.2016 Gazette 2016/06)**

(54) **PREPARATION METHOD OF DRUG-CONTAINING SUSTAINED RELEASE MICROPARTICLES**

VERFAHREN ZUR HERSTELLUNG WIRKSTOFFHALTIGER MIKROPARTIKEL MIT VERZÖGERTER FREISETZUNG

PROCÉDÉ DE PRÉPARATION DE MICROPARTICULES À LIBÉRATION PROLONGÉE CONTENANT UN MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2014 KR 20140102640**

(43) Date of publication of application:
**14.06.2017 Bulletin 2017/24**

(73) Proprietor: **Bcworld Pharm. Co., Ltd.**
**Ganam-myeon**
**Yeoju-gun, Gyeonggi-do 469-884 (KR)**

(72) Inventors:
• **SUH, Hea Ran**
  **Seoul 137-926 (KR)**
• **AN, Tae Kun**
  **Yongin-si**
  **Gyeonggi-do 446-916 (KR)**
• **CHOI, Ju Huen**
  **Seoul 134-734 (KR)**
• **OH, Seung Youn**
  **Cheonan-si**
  **Chungcheongnam-do 331-795 (KR)**
• **KIM, A Ram**
  **Gwangyang-si**
  **Jeollanam-do 545-804 (KR)**
• **KIM, Jong Min**
  **Suwon-si**
  **Gyeonggi-do 441-390 (KR)**
• **AN, Gug Hwan**
  **Gunpo-si**
  **Gyeonggi-do 435-010 (KR)**
• **NAM, Yoon Jin**
  **Yongin-si**
  **Gyeonggi-do 448-806 (KR)**
• **JEON, Dae Yeon**
  **Gunsan-si**
  **Jeollabuk-do 573-350 (KR)**
• **OH, Young Heun**
  **Seongnam-si**
  **Gyeonggi-do 463-853 (KR)**
• **HAN, Sang Min**
  **Suwon-si**
  **Gyeonggi-do 443-801 (KR)**
• **BAE, Min Hee**
  **Seongnam-si**
  **Gyeonggi-do 461-853 (KR)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
EP-A1- 1 629 833        WO-A1-99/03452
WO-A2-2004/064752       US-A- 5 656 299
US-A1- 2005 079 224     US-A1- 2005 276 859
US-A1- 2009 162 407     US-A1- 2012 083 444

**(Cont. next page)**

EP 3 178 471 B1

- **SONG KEDONG ET AL: "Fabrication and evaluation of a sustained-release chitosan-based scaffold embedded with PLGA microspheres", MATERIALS SCIENCE AND ENGINEERING C, ELSEVIER SCIENCE S.A, CH, vol. 33, no. 3, 23 December 2012 (2012-12-23), pages 1506-1513, XP028968679, ISSN: 0928-4931, DOI: 10.1016/J.MSEC.2012.12.054**

**Description**

TECHNICAL FIELD

The present disclosure relates to a preparation method of drug-containing sustained release microparticles.

BACKGROUND ART

Drugs that require administration for a long term have been used in various formulations. To reduce the number of administration and provide stable drug concentration in bodies, such drugs are preferably provided as sustained release preparations. A type of sustained release preparation is biodegradable microparticles containing drugs encapsulated in microspheres. An example is biodegradable microparticles containing luteinizing hormone-releasing hormone (leuprolide or LHRH), LUPRON Depot. Leuprolide is used to treat hormone-dependent cancers, in particular, prostate cancers and precocious puberty.

Microparticles are particles having the diameter of about 1 to 1000 microns. For injection, microparticles are preferably 125 microns or less in diameter. These sized microparticles can be injected via a standard hypodermic syringe needle instead of being surgically implanted. A type of microparticles is composed of a biodegradable polymer network which traps a drug. As the biodegradable polymer biodegrades in bodies, the drug is released. The most commonly used biodegradable polymer is polylactic acid, or lactic acid-glycolic acid copolymer.

The most widely used method for manufacturing sustained release microparticles is a phase separation method, a spray drying method, and a solvent evaporation method. The phase separation method, also known as coacervation, reduces the solubility of a polymer with the addition of a non-solvent. A general method for manufacturing microparticles dissolves a biodegradable polymer in an organic solvent such as dichloromethane. Lipophilic drugs are dissolved in the biodegradable polymer solution. Hydrophilic drugs are dissolved in water and then dispersed in the biodegradable polymer solution (W/O emulsion) or dispersed as solid powder. A non-solvent (usually, silicone oil) is slowly added to form two phases; namely, a silicone oil phase containing a polymer and an organic solvent phase free of a polymer. When the organic solvent is extracted or evaporated, the biodegradable polymer microparticles containing the drug are solidified in the silicone oil phase. Coacervate (silicone oil) is adsorbed to the polymer microparticles.

In the spray drying method, a biodegradable polymer is dissolved in a volatile organic solvent such as dichloromethane. A drug is dissolved or dispersed in the biodegradable polymer solution. The solution or dispersion is sprayed in heated air. The solvent is evaporated to form solid microparticles.

The solvent evaporation method is the most commonly used to manufacture microparticles. The solvent evaporation method emulsifies an organic polymer solution containing a drug in a dispersion medium, and the dispersion medium is generally water soluble but may be oil. The method can be sub-classified into an O/W method, a W/O/W method, and an O/O method.

In the O/W method, a drug and a polymer are dissolved in an organic solvent such as dichloromethane or a methanol/dichloromethane mixture. The drug-polymer-organic solvent solution is dispersed in an aqueous phase. To help organic solvent droplets to be formed in the aqueous phase, an emulsifier (usually, a surfactant) is added to the aqueous phase. The organic solvent is evaporated through stirring, and the droplets are solidified into drug-loaded polymer microparticles.

In the W/O/W method, to make a W/O emulsion including a drug and an organic solvent, a water-soluble drug solution is prepared and then dispersed in a solution of polymer in an organic solvent. To form a W/O/W emulsion, the W/O polymer-drug emulsion is emulsified in an aqueous phase. The organic solvent is evaporated with stirring, and polymer-drug droplets in the emulsion are solidified into microparticles.

In the O/O method, a drug and a polymer are dissolved in a solvent (for example, acetonitrile) that is mixed with water. To prepare an O/O emulsion, the solution is emulsified in an oil phase in the presence of an emulsifier such as SPAN 80. The organic solvent is extracted by oil and filtered to obtain microparticles.

PCT/JP1993/01673 (WO 94/10982) discloses an antipsychotic drug-containing sustained release microsphere preparation method using an O/W method of solvent evaporation. Specifically, the patent literature discloses a sustained release microsphere preparation method using a halogenated alkane solvent, for example, dichloromethane, but when halogenated alkane is used as a solvent, the halogenated alkane solvent may remain in microspheres composed of a biodegradable polymer and a drug, which is not easy to remove. The residual halogenated alkane solvent remaining in the microspheres may cause toxicity and cancer, and this is one of the important problem to be solved in the preparation of sustained release microspheres for administration into bodies.

To remove the residual halogenated alkane solvent, it is general to apply heat during preparation of sustained release microspheres, but when heated, polyester-based biodegradable polymers such as PLGA is subjected to accelerated hydrolysis and have a rapid reduction in molecular weight, causing the problem with attenuation of sustained drug release. Accordingly, there is the demand for preparation methods of sustained release microparticles that minimizes hydrolysis of a biodegradable polymer along with easily removing a halogenated alkane solvent.

## DISCLOSURE

### Technical Problem

The present disclosure is directed to providing a preparation method of drug-containing sustained release microparticles using a solvent evaporation method that minimizes molecular weight reduction of a biodegradable polymer in the microparticles while easily removing a halogenated alkane solvent remaining in the microparticles, thereby maintaining superior drug release.

### Technical Solution

As means for solving the problem, the present disclosure provides a preparation method of drug-containing sustained release microparticles including:

(a) dissolving a biodegradable polymer and a drug in a halogenated alkane solvent to form a drug-containing biodegradable polymer solution;
(b) homogeneously mixing the drug-containing biodegradable polymer solution in a continuous phase containing surfactant to form a dispersed phase;
(c) maintaining an emulsion including the continuous phase and the dispersed phase at temperature lower than the boiling point of the halogenated alkane solvent to form microparticles in the continuous phase;
(d) primarily drying the microparticles;
(e) mixing the primarily dried microparticles with an aqueous alcohol solution, and maintaining the aqueous alcohol solution at temperature above the boiling point of the halogenated alkane solvent to extract and evaporate the residual halogenated alkane solvent from the microparticles; and
(f) secondarily drying the obtained microparticles to produce drug-containing microparticles.

### Advantageous Effects

The preparation method of drug-containing sustained release microparticles of the present disclosure can prepare drug-containing sustained release microparticles with superior drug release by minimizing molecular weight reduction caused by hydrolysis of the biodegradable polymer in the microparticles, while easily removing the halogenated alkane solvent remaining in the microparticles.

### BEST MODE

The present disclosure relates to a preparation method of drug-containing sustained release microparticles including (a) dissolving a biodegradable polymer and a drug in a halogenated alkane solvent to form a drug-containing biodegradable polymer solution; (b) homogeneously mixing the drug-containing biodegradable polymer solution in a continuous phase containing surfactant to form a dispersed phase; (c) maintaining an emulsion including the continuous phase and the dispersed phase at temperature lower than the boiling point of the halogenated alkane solvent to form microparticles in the continuous phase; (d) primarily drying the microparticles; (e) mixing the primarily dried microparticles with an aqueous alcohol solution, and maintaining the aqueous alcohol solution at temperature above the boiling point of the halogenated alkane solvent to extract and evaporate the residual halogenated alkane solvent from the microparticles; and (f) secondarily drying the obtained microparticles to produce drug-containing microparticles.

Hereinafter, the preparation method of drug-containing sustained release microparticles of the present disclosure is described in detail.

The term "solvent evaporation method" as used herein refers to a method that dissolves a biodegradable polymer and a drug in an organic solvent to prepare a drug-containing biodegradable polymer solution, adds the drug-containing biodegradable polymer solution to a continuous phase containing surfactant, yielding microparticles, adds the microparticles to an aqueous alcohol solution to form drug-containing microparticles, and removes the residual organic solvent from the drug-containing microparticles.

The preparation method of the present disclosure includes (a) dissolving a biodegradable polymer and a drug in a halogenated alkane solvent to form a drug-containing biodegradable polymer solution.

The step (a) may be performed by a method that dissolves a biodegradable polymer in a halogenated alkane solvent to prepare a biodegradable polymer solution, and dissolves or disperses a drug in the biodegradable polymer solution to form a drug-containing biodegradable polymer solution, or a method that dissolves a biodegradable polymer and a drug in a halogenated alkane solvent at the same time to form a drug-containing biodegradable polymer solution.

The weight average molecular weight of the biodegradable polymer is not particularly limited, but its lower limit may be 10,000 or above, preferably 30,000 or above, more preferably 50,000 or above, and even more preferably 75,000 or above, and its upper limit may be 500,000 or less, preferably 400,000 or less, more preferably 300,000 or less, and even more preferably 200,000 or less.

The type of the biodegradable polymer is not particularly limited, but the biodegradable polymer may be preferably polyester, in particular, selected from the group consisting of polycaprolactone, lactic acid-caprolactone copolymer, polylactic acid, polyglycolic acid, lactic acid-glycolic acid copolymer and mixtures thereof, and more preferably lactic acid-glycolic acid copolymer.

When the lactic acid-glycolic acid copolymer is used as the biodegradable polymer, a molar ratio of lactic acid and glycolic acid in the copolymer may be 99:1 to 50:50, and preferably 75:25. Examples of commercial biodegradable polymers available to the present disclosure include Evonik Resomer RG 755S, Resomer RG756S and Resomer RG 858S.

The type of the drug used in the step (a) is not particularly limited, and the drug may be, for example, selected from the group consisting of antipsychotic drugs such as anti-cancer drugs; antianxiety drugs, antidepressant drugs, tranquilizing drugs and antipsychotic drugs; drugs for treatment of diseases of the cardiovascular system such as antihyperlipidemic drugs, antihypertensive drugs, antihypotensive drugs, antithrombotic drugs, vasodilating drugs, and antiarrhythmic drugs; antiepileptic drugs; drugs for treatment of diseases of the gastrointestinal system such as antiulcer drugs; anti-rheumatic drugs; antispasmodic drugs; antituberculosis drugs; muscle relaxant drugs; osteoporosis drugs; erectile dysfunction drugs; hemostatic drugs; hormone drugs such as sex hormone drugs; antidiabetic drugs; antibiotic drugs; antifungal drugs; antiviral drugs; antipyretic, anti-inflammatory and analgesic drugs; autonomic nervous modulating drugs; corticosteroid; diuretic drugs; antidiuretic drugs; analgesic drugs; anesthetic drugs; antihistaminic drugs; antiprotozoal drugs; antianemic drugs; antiasthmatic drugs; convulsion or spasm inhibitory drugs; antidotes; antimigraine drugs; antiemetic drugs; antiparkinson drugs; anticonvulsant drugs; antiplatelet drugs; antitussive drugs; bronchodilating drugs; cardiac stimulant drugs; immunomodulating drugs; protein drugs; gene drugs; and mixtures thereof, and preferably, may be selected from the group consisting of anti-cancer drugs, antipsychotic drugs, antihyperlipidemic drugs, antihypertensive drugs, antiepileptic drugs, drugs for treatment of diseases of the gastrointestinal system, anti-rheumatic drugs, antispasmodic drugs, antituberculosis drugs, muscle relaxant drugs, antiarrhythmic drugs, osteoporosis drugs, erectile dysfunction drugs, hemostatic drugs, antiviral drugs, hormone drugs, antibiotic drugs, antidiabetic drugs, antifungal drugs, antithrombotic drugs, antipyretic, anti-inflammatory and analgesic drugs, and mixtures thereof.

Among the enumerated drugs, the type of the antipsychotic drugs is not particularly limited, but may be preferably selected from the group consisting of haloperidol, bromperidol, fluphenazine maleate, chlorpromazine, chlorpromazine hibenzate, sulpiride, carpipramine hydrochloride, carpipramine maleate, clocapramine hydrochloride, mosapramine hydrochloride, risperidone, clozapine, olanzapine, sertindole and mixtures thereof, and more preferably, may be risperidone or its acid addition salt.

The halogenated alkane used as a solvent for dissolving the biodegradable polymer and the drug in the step (a) preferably has the boiling point of 120°C or less, and is immiscible with water. With the property of the halogenated alkane solvent that is immiscible with water, the drug-containing biodegradable polymer solution may be homogeneously mixed in a continuous phase containing surfactant to form a dispersed phase or a discontinuous phase in the step (b) as described below.

The type of the halogenated alkane solvent used in the step (a) is not particularly limited, but may be preferably chlorinated alkane, more preferably selected from the group consisting of dichloromethane, chloroform, chloroethane, dichloroethane, trichloroethane and mixtures thereof, and most preferably dichloromethane.

The preparation method of the present disclosure includes (b) homogeneously mixing the drug-containing biodegradable polymer solution in a continuous phase containing surfactant to form a dispersed phase.

The method of homogeneously mixing the drug-containing biodegradable polymer solution with a continuous phase containing surfactant in the step (b) is not particularly limited, but may be preferably performed using a high speed stirrer.

In the case of forming an emulsion including the continuous phase and the dispersed phase as in the step (b), when the drug-containing biodegradable polymer solution is homogeneously dispersed in a continuous phase containing surfactant, a dispersed phase or a discontinuous phase is formed in the form of droplets.

The type of the surfactant used in the step (b) is not particularly limited, but includes any type of surfactant that helps the drug-containing biodegradable polymer solution to form a dispersed phase of droplets that are stable in the continuous phase, to form an O/W (Oil in Water) emulsion. The surfactant may be preferably selected from the group consisting of non-ionic surfactants such as methylcellulose, polyvinylpyrrolidone, carboxymethylcellulose, lecithin, gelatin, polyvinylalcohol, polyoxyethylene sorbitan fatty acid ester and polyoxyethylene castor oil derivatives; anionic surfactants such as sodium lauryl sulfate and sodium stearate; cationic surfactants such as imidazole, ester amine, linear diamine and fatty amine, and mixtures thereof, and the most preferably polyvinylalcohol.

5

In the step (b), an amount of surfactant in the continuous phase containing the surfactant may be 0.01 w/v% to 20 w/v%, and preferably 0.1 w/v% to 5 w/v%, per the total volume of the continuous phase containing the surfactant. When the amount of surfactant is less than 0.01 w/v%, a dispersed phase or a discontinuous phase in the form of droplets may not be formed in the continuous phase, and when the amount of surfactant is more than 20 w/v%, it may be difficult to remove the surfactant after microparticles are formed in the continuous phase due to the surfactant in excessive.

The continuous phase used in the step (b) may be water. When water is used as the continuous phase, after microparticles are formed in the continuous phase in the step (c) as described below, the continuous phase and the water-soluble surfactant such as polyvinylalcohol remaining on the microparticle surface can be removed more easily through filtration and washing before performing the step (d) as described below.

The continuous phase containing surfactant used in the step (b) may be preferably a polyvinyl alcohol aqueous solution. That is, polyvinylalcohol may be used as the surfactant, and water may be used as the continuous phase.

The preparation method of the present disclosure includes (c) maintaining an emulsion including the dispersed phase and continuous phase formed in the step (b) at the temperature lower than the boiling point of the halogenated alkane solvent to form microparticles in the continuous phase.

In the step (c), when an emulsion including the drug-containing biodegradable polymer solution in the form of droplets (a dispersed phase or discontinuous phase) and the continuous phase containing surfactant is maintained or stirred at temperature lower than the boiling point of the halogenated alkane solvent for a predetermined period of time, for example, 2 hours to 3 hours, the halogenated alkane solvent may be extracted from the dispersed phase or discontinuous phase, namely, the drug-containing biodegradable polymer solution in the form of droplets towards the continuous phase. As the halogenated alkane solvent is extracted from the drug-containing biodegradable polymer solution in the form of droplets, the dispersed phase or discontinuous phase in the form of droplets are solidified to form microparticles.

The conventional preparation method of microparticles using a solvent evaporation method applies heat to remove a halogenated alkane solvent from a dispersed phase or discontinuous phase in the form of droplets, and by the heat, hydrolysis of a biodegradable polymer occurs, causing a molecular weight reduction problem.

However, in the present disclosure, because the step (c) is performed at temperature lower than the boiling point of the halogenated alkane solvent, the dispersed phase or discontinuous phase in the form of droplets is solidified while minimizing hydrolysis of the biodegradable polymer by heat, and thus, microparticles containing the biodegradable polymer and the drug are formed in the continuous phase.

The preparation method of the present disclosure may further include, between the step (c) and the step (d) as described below, removing the continuous phase through filtration and washing to obtain microparticles.

As a result of performing the step (c), the microparticles composed of the biodegradable polymer, the drug and the residual halogenated alkane solvent are dispersed in the continuous phase to produce a suspension. In the suspension, the liquid continuous phase, the surfactant, the extracted halogenated alkane solvent, and the dispersed microparticles are present. Accordingly, before performing the drying step (d) as described below, the suspension is filtered to obtain solidified microparticles, and the solidified microparticles are washed with water at least once, and preferably one to three times to remove the surfactant, and are filtered again to obtain the washed microparticles.

The washing step for removing the residual surfactant may be generally performed using water used as the continuous phase, and the washing step may be iteratively performed a few times.

The preparation method of the present disclosure includes, after the step (d) or after the filtering and washing steps, primarily drying the microparticles.

In the step (d), the primary drying method is not particularly limited, but may be preferably performed through vacuum drying to minimize damage of the biodegradable polymer by heat. When vacuum drying is performed on the microparticles obtained through filtration and washing, moisture present in the microparticles may be removed through evaporation or sublimation. The vacuum drying may be preferably performed at room temperature.

As a result of performing the step (d), the solidified microparticles obtained by the primary drying has a solid film formed thereon, thereby preventing the hydrolysis of the biodegradable polymer by heat even though the temperature of the aqueous alcohol solution is maintained above the boiling point of the halogenated alkane solvent in the step (e) as described below.

The preparation method of the present disclosure includes (e) mixing the primarily dried microparticles obtained in the step (d) with an aqueous alcohol solution, and maintaining the aqueous alcohol solution at temperature above the boiling point of the halogenated alkane solvent to extract and evaporate the residual halogenated alkane solvent from the microparticles.

Besides the biodegradable polymer and the drug, the halogenated alkane solvent remains in the primarily dried microparticles obtained in the step (d), so there is the need to remove the residual solvent from the microparticles.

Accordingly, when the primarily dried microparticles obtained in the step (d) are mixed with an aqueous alcohol solution, and maintained or stirred at temperature above the boiling point of the halogenated alkane solvent for a predetermined period of time, 2 hours to 3 hours, the halogenated alkane solvent remaining in the microparticles is dissolved in the aqueous alcohol solution and is extracted and evaporated.

The type of alcohol used in the step (e) is not particularly limited, but may be preferably selected from the group consisting of methanol, ethanol, isopropanol and mixtures thereof, and more preferably, may be ethanol.

The alcohol content in the aqueous alcohol solution may be 5 volume% to 80 volume%, and preferably 10 volume% to 50 volume%, per the total volume of the aqueous alcohol solution. When the alcohol content is less than 5 volume%, extraction of the halogenated alkane solvent remaining in the microparticles is not easy and the process time may increase, and when the alcohol content is more than 80 volume%, agglomeration between the microparticles may occur.

The preparation method of the present disclosure may further include, between the step (e) and the step (f) as described below, removing the aqueous alcohol solution through filtration and washing to obtain microparticles.

As a result of performing the step (e), the aqueous alcohol solution includes alcohol; water; and the microparticles composed of the biodegradable polymer and the drug. Accordingly, before performing the drying step (f) as described below, the aqueous alcohol solution may be filtered to obtain solidified microparticles, and the solidified microparticles may be washed with water at least once, and preferably one to three times, to remove alcohol, and filtered again to obtain the washed microparticles.

The washing step to remove alcohol may be performed using water used as the continuous phase, and the washing step may be iteratively performed a few times.

The preparation method of the present disclosure includes (f) secondarily drying the obtained microparticles to produce drug-containing microparticles after the step (e) or the filtering and washing steps.

In the step (f), the second drying method is not particularly limited, but may be preferably performed through vacuum drying to minimize damage of the biodegradable polymer by heat. When vacuum drying is performed on the microparticles obtained through filtration and washing, water, alcohol remaining after washing and the halogenated alkane solvent remaining in water may be removed from the microparticles through evaporation or sublimation. The vacuum drying may be preferably performed at room temperature.

As a result of performing the step (f), the drug-containing sustained release microparticles composed of the biodegradable polymer and the drug may be obtained.

Because the halogenated alkane solvent is absent in the drug-containing sustained release microparticles prepared according to the preparation method of the present disclosure, the problem with toxicity and cancer that is problematic when the halogenated alkane solvent remains can be solved, and in the process of removing the halogenated alkane solvent remaining in the microparticles, hydrolysis of the biodegradable polymer can be minimized, thereby maintain the sustained drug release of the microparticles.

In the preparation of drug-containing microparticles using a solvent evaporation method as in the prior art, when the molecular weight reduces due to hydrolysis of a biodegradable polymer in the microparticles, hydrolysis of the biodegradable polymer is accelerated due to the reduced molecular weight when administering the prepared microparticles into bodies, failing to achieve a sustained drug release effect by which a drug is slowly released.

However, when drug-containing sustained release microparticles are prepared according to the preparation method of the present disclosure, minimized hydrolysis of a biodegradable polymer can prevent the reduction in molecular weight, thereby achieving a superior drug release effect when administering the microparticles into bodies.

The drug-containing sustained release microparticles prepared according to the preparation method of the present disclosure may include 20 wt% to 99 wt%, and preferably 50 wt% to 80 wt% of biodegradable polymer per the total weight of the microparticles.

MODE FOR CARRYING OUT THE INVENTION

### Example 1

10g PLGA (lactic acid-glycolic acid copolymer, a molar ratio of lactic acid and glycolic acid = 75:25) as a biodegradable polymer and 8g risperidone as a drug was dissolved in 40ml dichloromethane (boiling point: 39.6°C) solvent to prepare a drug-containing biodegradable polymer solution. Furthermore, 5L polyvinyl alcohol aqueous solution (concentration: 0.25 w/v%) was prepared using polyvinylalcohol as a surfactant and water as a continuous phase. Subsequently, the drug-containing biodegradable polymer solution was mixed with the polyvinyl alcohol aqueous solution and stirred at a high speed to form a dispersed phase in the form of droplets. Furthermore, an emulsion including the continuous phase and the dispersed phase was maintained at 15°C for 2 to 3 hours, to allow the dichloromethane solvent to be extracted from the drug-containing biodegradable polymer solution in the form of droplets towards the polyvinyl alcohol aqueous solution, followed by solidification of the droplets, through which microparticles were formed in the continuous phase. Subsequently, the solidified microparticles were filtered and washed with water three times to remove polyvinylalcohol. Subsequently, filtering was performed again, through which the washed microparticles were obtained, and primarily dried under vacuum to evaporate moisture and dichloromethane remaining in the microparticles, yielding the primarily dried microparticles. The primarily dried microparticles were mixed with 500mL aqueous ethanol solution (concentration: 25v/v%) and stirred at 45°C for 2 to 3 hours to extract dichloromethane remaining in the microparticles towards the aqueous ethanol solution, allowing the dichloromethane to evaporate. Subsequently, the aqueous ethanol solution including the microparticles was filtered, and the microparticles were washed with water three times to remove ethanol. Subsequently, filtering was performed again, through which the washed microparticles were obtained, and secondarily dried under vacuum to remove moisture, dichloromethane and ethanol remaining in the microparticles, yielding drug-containing sustained release microparticles.

## Comparative example 1

A drug-containing biodegradable polymer solution and 5L polyvinyl alcohol aqueous solution were prepared by the same method as example 1. Subsequently, the drug-containing biodegradable polymer solution was mixed with the polyvinyl alcohol aqueous solution and stirred at a high speed to form a dispersed phase in the form of droplets. Furthermore, an emulsion including the continuous phase and the dispersed phase was maintained at 15°C for 2 to 3 hours, to allow the dichloromethane solvent to be extracted from the drug-containing biodegradable polymer solution in the form of droplets towards the polyvinyl alcohol aqueous solution, followed by solidification of the droplets, through which microparticles were formed in the continuous phase. Subsequently, the continuous phase including the microparticles was stirred at 45°C for 2 to 3 hours to evaporate dichloromethane from the microparticles, and the solidified microparticles were filtered and washed with water three times to remove the polyvinylalcohol. Subsequently, filtering was performed again to obtain the washed microparticles which were dried under vacuum to evaporate moisture and dichloromethane remaining in the microparticles, yielding drug-containing microparticles.

## Comparative example 2

A drug-containing biodegradable polymer solution and 5L polyvinyl alcohol aqueous solution were prepared by the same method as example 1. Subsequently, the drug-containing biodegradable polymer solution was mixed with the polyvinyl alcohol aqueous solution and stirred at a high speed to form a dispersed phase in the form of droplets. Furthermore, an emulsion including the continuous phase and the dispersed phase was maintained at 15°C for 2 to 3 hours, to allow the dichloromethane solvent to be extracted from the drug-containing biodegradable polymer solution in the form of droplets towards the polyvinyl alcohol aqueous solution, followed by solidification of the droplets, through which microparticles were formed in the continuous phase. Subsequently, the continuous phase including the microparticles was stirred at 45°C for 2 to 3 hours to evaporate dichloromethane from the microparticles, so the microparticles were further solidified. Subsequently, the solidified microparticles were filtered and washed with water three times to remove polyvinylalcohol. Subsequently, filtering was performed again, through which the washed microparticles were obtained, and primarily dried under vacuum to evaporate moisture and dichloromethane remaining in the microparticles, yielding the primarily dried microparticles. The primarily dried microparticles were mixed with 500mL aqueous ethanol solution (concentration: 25v/v %), and stirred at 45°C for 2 to 3 hours to extract residual dichloromethane from the microparticles towards the aqueous ethanol solution, allowing the dichloromethane to evaporate. Subsequently, the aqueous ethanol solution including the microparticles was filtered, and the microparticles were washed with water three times to remove ethanol. Subsequently, filtering was performed again, through which the washed microparticles were obtained, and secondarily dried under vacuum to remove moisture, dichloromethane and ethanol remaining in the microparticles, yielding drug-containing microparticles.

## Test example 1: Measurement of weight average molecular weight of biodegradable polymer in microparticles

The weight average molecular weight of the biodegradable polymer (i.e., PLGA) in the drug-containing microparticles obtained in example 1 and comparative examples 1 and 2 was measured using high-performance liquid chromatography (Waters, GPC-150C Plus) as follows. Fractionation columns were connected in series in the order of Shodex GPC KF-G, 804, 803, 802 and 801. The column temperature was set to 50°C, the flow rate was 1.0 ml/min using tetrahydrofuran as mobile phase, and a differential refractometer was used as a detector. Subsequently, the weight average molecular weight of a test solution was calculated using weight average molecular weight calibration curves of each polystyrene standard solution.

## Test example 2: Measurement of an amount of solvent remaining in microparticles

Measurements of an amount of solvent remaining in the drug-containing microparticles obtained in example 1 and comparative examples 1 and 2 were performed by correctly transfering 5ml of each of the test solution and the standard solution to headspace vials. Peak areas AT and AS of dichloromethane and ethanol in each of the test solution and the standard solution were measured and an amount of residual solvents in the obtained microparticles was calculated by the following equation.

$$\text{Amount of residual dichloromethane in microparticles (ppm)} =$$

$$\frac{AT}{AS} \times \frac{WS}{WT} \times \frac{5}{20000} \times 1000000$$

AT: Peak area of dichloromethane and ethanol in test solution

AS: Peak area of dichloromethane and ethanol in standard solution

WT: Harvested amount of obtained microparticles (g)

WS: Harvested amount of standard foam (g)

The test example measurement results of example 1 and comparative examples 1 and 2 are summarized in the following Table 1.

[Table 1]

|  |  | Amount of residual dichloromethane in microparticles (ppm) | Weight average molecular weight of biodegradable polymer in microparticles |
|---|---|---|---|
| Example | 1 | 0 | 139,876 |
| Comparative example | 1 | 9222 | 127,588 |
|  | 2 | 0 | 88,963 |

As shown in Table 1, in the case of example 1, an emulsion including the continuous phase and the dispersed phase was maintained at temperature lower than the boiling point of the halogenated alkane solvent to form microparticles in the continuous phase, and the microparticles obtained by primary vacuum drying were mixed with an aqueous alcohol solution and maintained at temperature above the boiling point of the halogenated alkane solvent to remove the halogenated alkane solvent remaining in the microparticles by extraction and evaporation, followed by secondary vacuum drying, to completely remove the halogenated alkane solvent remaining in the microparticles while minimizing hydrolysis of the biodegradable polymer in the microparticles.

However, in the case of comparative example 1, dissimilar to example 1, the continuous phase including microparticles at temperature above the boiling point of the halogenated alkane solvent was maintained without primary vacuum drying and additional removal of the halogenated alkane solvent by the aqueous alcohol solution, to further solidify the microparticles, followed by vacuum drying to obtain microparticles, and it can be seen that the halogenated alkane solvent remaining in the microparticles is present in a large amount.

Furthermore, in the case of comparative example 2, dissimilar to example 1, before primary vacuum drying, the step for maintaining the continuous phase including microparticles at temperature above the boiling point of the halogenated alkane solvent to further solidify the microparticles was performed, and thus, the halogenated alkane solvent remaining in the microparticles was completely removed, but due to this, hydrolysis of the biodegradable polymer in the microparticles occurred, and it can be seen that the biodegradable polymer reduced in weight average molecular weight.

As described hereinabove, when drug-containing sustained release microparticles are prepared according to the preparation method of the present disclosure, the halogenated alkane solvent remaining in the microparticles can be completely removed, and in this process, molecular weight reduction resulting from hydrolysis of the biodegradable polymer in the microparticles can be minimized. Accordingly, the drug-containing sustained release microparticles prepared according to the preparation method of the present disclosure can completely remove the halogenated alkane solvent that is toxic and cause cancers, while maintaining the weight average molecular weight of the biodegradable polymer, thereby maintaining superior sustained drug release.

**Claims**

1. A preparation method of drug-containing sustained release microparticles, comprising:

   (a) dissolving a biodegradable polymer and a drug in a halogenated alkane solvent to form a drug-containing biodegradable polymer solution;
   (b) homogeneously mixing the drug-containing biodegradable polymer solution in a continuous phase containing surfactant to form a dispersed phase;
   (c) maintaining an emulsion including the continuous phase and the dispersed phase at temperature lower than the boiling point of the halogenated alkane solvent to form microparticles in the continuous phase;
   (d) primarily drying the microparticles;
   (e) mixing the primarily dried microparticles with an aqueous alcohol solution, and maintaining the aqueous alcohol solution at temperature above the boiling point of the halogenated alkane solvent to extract and evaporate the residual halogenated alkane solvent from the microparticles; and
   (f) secondarily drying the obtained microparticles to produce drug-containing microparticles.

2. The preparation method of drug-containing sustained release microparticles according to claim 1, wherein the biodegradable polymer is selected from the group consisting of polycaprolactone, lactic acid-caprolactone copolymer, polylactic acid, polyglycolic acid, lactic acid-glycolic acid copolymer, and mixtures thereof.

3. The preparation method of drug-containing sustained release microparticles according to claim 1, wherein the drug is selected from the group consisting of anti-cancer drugs, antipsychotic drug, antihyperlipidemic drugs, antihypertensive drugs, antiepileptic drugs, drugs for treatment of diseases of the gastrointestinal system, anti-rheumatic drugs, antispasmodic drugs, antituberculosis drugs, muscle relaxant drugs, antiarrhythmic drugs, osteoporosis drugs, erectile dysfunction drugs, hemostatic drugs, antiviral drugs, hormone drugs, antibiotic drugs, antidiabetic drugs, antifungal drugs, antithrombotic drugs, antipyretic, anti-inflammatory and analgesic drugs, and mixtures thereof.

4. The preparation method of drug-containing sustained release microparticles according to claim 3, wherein the antipsychotic drug is selected from the group consisting of haloperidol, bromperidol, fluphenazine maleate, chlorpromazine, chlorpromazine hibenzate, sulpiride, carpipramine hydrochloride, carpipramine maleate, clocapramine hydrochloride, mosapramine hydrochloride, risperidone, clozapine, olanzapine, sertindole, and mixtures thereof.

5. The preparation method of drug-containing sustained release microparticles according to claim 1, wherein the halogenated alkane solvent is selected form the group consisting of dichloromethane, chloroform, chloroethane, dichloroethane, trichloroethane, and mixtures thereof.

6. The preparation method of drug-containing sustained release microparticles according to claim 1, wherein the surfactant is selected from the group consisting of a nonionic surfactant, an anionic surfactant, a cationic surfactant, and mixtures thereof.

7. The preparation method of drug-containing sustained release microparticles according to claim 1, wherein the continuous phase containing surfactant is a polyvinyl alcohol aqueous solution.

8. The preparation method of drug-containing sustained release microparticles according to claim 1, wherein the alcohol is selected from the group consisting of methanol, ethanol, isopropanol, and mixtures thereof.

9. The preparation method of drug-containing sustained release microparticles according to claim 1, wherein the primary drying and the secondary drying are performed by vacuum drying.

10. The preparation method of drug-containing sustained release microparticles according to claim 1, further comprising:

    between the step (c) and the step (d),
    removing the continuous phase through filtration and washing to obtain microparticles.

11. The preparation method of drug-containing sustained release microparticles according to claim 1, further comprising:

    between the step (e) and the step (f),

removing the aqueous alcohol solution through filtration and washing to obtain microparticles.

**12.** The preparation method of drug-containing sustained release microparticles according to claim 1, wherein the drug-containing microparticles comprise 20 wt% to 99 wt% of biodegradable polymer per the total weight of the drug-containing microparticles.

**Patentansprüche**

**1.** Herstellungsverfahren von arzneimittelhaltigen Mikropartikeln mit verzögerter Freisetzung, umfassend:

(a) Lösen eines biologisch abbaubaren Polymers und eines Arzneimittels in einem halogenierten Alkanlösungsmittel, um eine arzneimittelhaltige biologisch abbaubare Polymerlösung zu bilden;
(b) homogenes Mischen der arzneimittelhaltigen biologisch abbaubaren Polymerlösung in einer kontinuierlichen Phase, die Tensid enthält, um eine dispergierte Phase zu bilden;
(c) Halten einer Emulsion, welche die kontinuierliche Phase und die dispergierte Phase beinhaltet, bei einer Temperatur, die niedriger ist als der Siedepunkt des halogenierten Alkanlösungsmittels, um Mikropartikel in der kontinuierlichen Phase zu bilden;
(d) primäres Trocknen der Mikropartikel;
(e) Mischen der primär getrockneten Mikropartikel mit einer wässrigen Alkohollösung und Halten der wässrigen Alkohollösung bei einer Temperatur oberhalb des Siedepunkts des halogenierten Alkanlösungsmittels, um das restliche halogenierte Alkanlösungsmittel aus den Mikropartikeln zu extrahieren und zu verdampfen; und
(f) sekundäres Trocknen der erhaltenen Mikropartikel, um arzneimittelhaltige Mikropartikel herzustellen.

**2.** Herstellungsverfahren von arzneimittelhaltigen Mikropartikeln mit verzögerter Freisetzung nach Anspruch 1, wobei das biologisch abbaubare Polymer ausgewählt ist aus der Gruppe bestehend aus Polycaprolacton, Milchsäure-Caprolacton-Copolymer, Polymilchsäure, Polyglykolsäure, Milchsäure-Glykolsäure-Copolymer und Mischungen davon.

**3.** Herstellungsverfahren von arzneimittelhaltigen Mikropartikeln mit verzögerter Freisetzung nach Anspruch 1, wobei das Arzneimittel ausgewählt ist aus der Gruppe bestehend aus Krebsarzneimitteln, antipsychotischen Arzneimitteln, antihyperlipidämischen Arzneimitteln, antihypertensiven Arzneimitteln, antiepileptischen Arzneimitteln, Arzneimitteln zur Behandlung von Erkrankungen des Magen-Darm-Systems, antirheumatischen Arzneimitteln, krampflösenden Arzneimitteln, Antituberkulosearzneimitteln, Muskelrelaxantien, Antiarrhythmika, Osteoporosearzneimitteln, Arzneimitteln gegen erektile Dysfunktion, Hämostatika, antiviralen Arzneimitteln, Hormonarzneimitteln, Antibiotika, Antidiabetika, Antimykotika, Arzneimitteln gegen Thrombosen, Antipyretika, entzündungshemmenden und schmerzstillenden Arzneimitteln und Mischungen davon.

**4.** Herstellungsverfahren von arzneimittelhaltigen Mikropartikeln mit verzögerter Freisetzung nach Anspruch 3, wobei das antipsychotische Arzneimittel ausgewählt ist aus der Gruppe bestehend aus Haloperidol, Bromperidol, Fluphenazinmaleat, Chlorpromazin, Chlorpromazinhibenzat, Sulpirid, Carpipraminhydrochlorid, Carpipraminmaleat, Clocapraminhydrochlorid, Mosapraminhydrochlorid, Risperidon, Clozapin, Olanzapin, Sertindole und Mischungen davon.

**5.** Herstellungsverfahren von arzneimittelhaltigen Mikropartikeln mit verzögerter Freisetzung nach Anspruch 1, wobei das halogenierte Alkanlösungsmittel ausgewählt ist aus der Gruppe bestehend aus Dichlormethan, Chloroform, Chlorethan, Dichlorethan, Trichlorethan und Mischungen davon.

**6.** Herstellungsverfahren von arzneimittelhaltigen Mikropartikeln mit verzögerter Freisetzung nach Anspruch 1, wobei das Tensid ausgewählt ist aus der Gruppe bestehend aus einem nichtionischen Tensid, einem anionischen Tensid, einem kationischen Tensid und Mischungen davon.

**7.** Herstellungsverfahren von arzneimittelhaltigen Mikropartikeln mit verzögerter Freisetzung nach Anspruch 1, wobei die kontinuierliche, tensidhaltige Phase eine wässrige Polyvinylalkohollösung ist.

**8.** Herstellungsverfahren von arzneimittelhaltigen Mikropartikeln mit verzögerter Freisetzung nach Anspruch 1, wobei der Alkohol ausgewählt ist aus der Gruppe bestehend aus Methanol, Ethanol, Isopropanol und Mischungen davon.

9.  Herstellungsverfahren von arzneimittelhaltigen Mikropartikeln mit verzögerter Freisetzung nach Anspruch 1, wobei das primäre Trocknen und das sekundäre Trocknen durch Vakuumtrocknung durchgeführt werden.

10. Herstellungsverfahren von arzneimittelhaltigen Mikropartikeln mit verzögerter Freisetzung nach Anspruch 1, ferner umfassend:

    zwischen dem Schritt (c) und dem Schritt (d),
    Entfernen der kontinuierlichen Phase durch Filtration und Waschen, um Mikropartikel zu erhalten.

11. Herstellungsverfahren von arzneimittelhaltigen Mikropartikeln mit verzögerter Freisetzung nach Anspruch 1, ferner umfassend:

    zwischen dem Schritt (e) und dem Schritt (f),
    Entfernen der wässrigen Alkohollösung durch Filtration und Waschen, um Mikropartikel zu erhalten.

12. Herstellungsverfahren von arzneimittelhaltigen Mikropartikeln mit verzögerter Freisetzung nach Anspruch 1, wobei die arzneimittelhaltigen Mikropartikel 20 Gew.-% bis 99 Gew.-% biologisch abbaubares Polymer pro Gesamtgewicht der arzneimittelhaltigen Mikropartikel umfassen.

**Revendications**

1.  Un procédé de préparation de microparticules à libération prolongée contenant un médicament, comprenant :

    a) dissoudre un polymère biodégradable et un médicament dans un solvant alcane halogéné pour former une solution polymère biodégradable contenant un médicament ;
    b) mélanger de façon homogène la solution de polymère biodégradable contenant le médicament dans une phase continue contenant un agent tensio-actif pour former une phase dispersée ;
    c) maintenir une émulsion comprenant la phase continue et la phase dispersée à une température inférieure au point d'ébullition du solvant alcane halogéné pour former des microparticules dans la phase continue ;
    d) sécher primairement les microparticules ;
    e) mélanger les microparticules primairement séchées avec une solution hydro-alcoolique, et maintenir la solution hydro-alcoolique à une température supérieure à la température d'ébullition du solvant alcane halogéné pour extraire et évaporer le solvant alcane halogéné résiduel des microparticules ; et
    f) sécher secondairement les microparticules obtenues pour produire les microparticules contenant le médicament.

2.  Le procédé de préparation de microparticules à libération prolongée contenant un médicament selon la revendication 1, dans lequel le polymère biodégradable est choisi dans le groupe consistant en polycaprolactone, copolymère d'acide lactique-caprolactone, acide polylactique, acide polyglycolique, copolymère d'acide lactique-acide glycolique et mélanges de ceux-ci.

3.  Le procédé de préparation de microparticules à libération prolongée contenant un médicament selon la revendication 1, dans lequel le médicament est choisi dans le groupe consistant en médicaments anticancéreux, médicaments antipsychotiques, médicaments antihyperlipidémiques, médicaments antihypertenseurs, médicaments antiépileptiques, médicaments antipsychotiques, médicaments pour le traitement des maladies du système gastrointestinal, médicaments antirhumatismaux, médicaments contre l'ostéoporose, médicaments contre le dysfonctionnnement érectile, médicaments hémostatiques, médicaments antiviraux, de médicaments hormonaux, médicaments antibiotiques, médicaments antidiabétiques, médicaments antifongiques, médicaments anti-thrombotiques, médicaments antipyrétiques, médicaments anti-inflammatoires et médicaments analgésiques, et mélanges de ceux-ci.

4.  Le procédé de préparation de microparticules à libération prolongée contenant un médicament selon la revendication 1, dans lequel le médicament antipsychotique est choisi dans le groupe consistant en l'halopéridol, le brompéridol, le maléate de fluphénazine, la chlorpromazine, l'hibenzate de chlorpromazine, le sulpiride, le chlorhydrate de carpipramine, le maléate de carpipramine, le chlorhydrate de clocapramine, le chlorhydrate de mosapramine, la rispéridone, la clozapine, l'olanzapine, le sertindole et leurs mélanges.

5.  Le procédé de préparation de microparticules à libération prolongée contenant un médicament selon la revendication 1, dans lequel le solvant alcane halogéné est choisi dans le groupe consistant en le dichlorométhane, le chloroforme, le chloroéthane, le dichloroéthane, le trichloroéthane et leurs mélanges.

6.  Le procédé de préparation de microparticules à libération prolongée contenant un médicament selon la revendication 1, dans lequel l'agent tensioactif est choisi dans le groupe consistant en un agent tensioactif non ionique, un agent tensioactif anionique, un agent tensioactif cationique et leurs mélanges.

7.  Le procédé de préparation de microparticules à libération prolongée contenant un médicament selon la revendication 1, dans lequel la phase continue contenant l'agent tensioactif est une solution hydro-alcoolique polyvinylique.

8.  Le procédé de préparation de microparticules à libération prolongée contenant un médicament selon la revendication 1, dans lequel l'alcool est choisi dans le groupe constitué par le méthanol, l'éthanol, l'isopropanol et leurs mélanges.

9.  Le procédé de préparation de microparticules à libération prolongée contenant un médicament selon la revendication 1, dans lequel le séchage primaire et le séchage secondaire sont réalisés par séchage sous vide.

10. Le procédé de préparation de microparticules à libération prolongée contenant un médicament selon la revendication 1, comprenant en outre :

    entre l'étape (c) et l'étape (d),
    élimination de la phase continue par filtration et lavage pour obtenir des microparticules.

11. Le procédé de préparation de microparticules à libération prolongée contenant un médicament selon la revendication 1, comprenant en outre :

    entre l'étape (e) et l'étape (f),
    élimination de la solution hydro-alcoolique par filtration et lavage pour obtenir des microparticules.

12. Le procédé de préparation de microparticules à libération prolongée contenant un médicament selon la revendication 1, dans lequel les microparticules contenant un médicament comprennent 20% en poids à 99% en poids de polymère biodégradable par rapport au poids total des microparticules contenant le médicament.

**EP 3 178 471 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5001673 W

- WO 9410982 A